# FASCICULE DE BREVET EUROPEEN

(11) **EP 0 296 001 B1**
(45) Date de publication et mention de la délivrance du brevet: **31.03.1993**
(21) Numéro de dépôt: 88401325.1
(22) Date de dépôt: 01.06.1988
(51) Int. Cl.: A61N 1/05, A61B 5/04

(54) **Perfectionnements aux extrémités conductrices de sondes de stimulation cardiaque**
Leitende Enden von Elektroden für Herzstimulation
Conducting extremites of cardiac stimulation electrodes

(30) Priorité: 04.06.1987 FR 8707788
(43) Date de publication de la demande: 21.12.1988
(73) Titulaire: ELA MEDICAL, F-92541 Montrouge (FR)
(72) Inventeur: Olivier, Stéphane, F-75015 Paris (FR)
(74) Mandataire: Loyer, Bertrand

(56) Documents cités:
- EP-A- 0 047 013
- EP-A- 0 084 973
- EP-A- 0 191 238
- FR-A- 2 345 170
- US-A- 4 566 467
- PRODUCT ENGINEERING, vol. 49, no. 5, mai 1978, page 14; "Pacemaker challenges design ingenuity"

## Description

La présente invention est relative à des perfectionnements aux extrémités conductrices de sondes de stimulation cardiaque.

Il est connu depuis de nombreuses années de réaliser la stimulation cardiaque au moyen d'un générateur de signaux électriques implanté en général sous la peau du thorax et relié à la paroi interne du coeur par un conducteur passant par une veine, par exemple , la veine sous clavière. Ce conducteur, appelé soit cathéter soit sonde endocardiaque, est constitué par un fil métallique, enroulé en spirale, disposé à l'intérieur d'une gaîne souple, par exemple en caoutchouc au silicone et terminé par une pièce conductrice destinée à être mise en contact avec la paroi interne du muscle cardiaque, cette pièce constituant l'électrode proprement dite.

Le document EP 0 191 238 décrit un exemple de sonde endocardiaque pour stimulateur cardiaque du type constitué par un conducteur enroulé en spirale, disposé à l'intérieur d'une gaîne souple et relié à une électrode. L'électrode s'engage partiellement à l'intérieur de la gaine souple et y est fixée par un adhésif à base de polyuréthane.

L'utilisation de nouvelles électrodes en matériaux inorganiques tels que les matériaux carbone (pyrolytique, vitreux etc...) ou les céramiques à plasticité nulle et faible tenacité demande un système de raccordement électrode-conducteur souple et fiable. Ce système de fixation réalise le plus souvent à la fois la liaison mécanique et la liaison électrique entre l'électrode et le conducteur. De plus il est souhaitable que le système choisi permette un montage facile en utilisant des éléments standard interchangeables permettant en particulier de placer des électrodes de matière, forme et surface différentes. Il est intéressant de pouvoir moduler la surface de l'électrode ; par exemple la réduire , ce qui augmente la densité de courant locale et conduit à une diminution des seuils de stimulation et de l'énergie nécessaire pour exiter le coeur.

La présente invention a pour objet une sonde endocardiaque pour stimulateur cardiaque, du type constitué par un conducteur enroulé en spirale, disposé à l'intérieur d'une gaine souple, à l'extrémité duquel est placée une électrode constituée par une pièce annulaire traversée par un organe de fixation comportant une tige s'engageant à l'intérieur du conducteur et une tête assurant le maintien de l'électrode, caractérisée en ce que : l'électrode est placée sur l'extrémité distale de la gaine souple et est maintenue par coincement entre le conducteur enroulé en spirale et la tête de l'organe de fixation.

Selon d'autres modes de réalisation particuliers de l'invention :
- la tête est recouverte d'une couche de matériau isolant ;
- la tête de l'organe de fixation constitue une butée dont la forme est complémentaire de l'orifice central de l'électrode annulaire ;
- la tête de l'organe de fixation est tronconique et s'adapte à la forme tronconique correspondante de l'orifice central de l'électrode annulaire ;
- la tête de l'organe de fixation est plate et prend appui sur un épaulement circulaire de forme correspondante ménagé dans l'orifice central de l'électrode annulaire ;
- une bague est sertie sur la tige de l'organe de fixation de sorte que l'électrode annulaire est bloquée entre la tête dudit organe de fixation et la bague ;
- l'organe de fixation est solidarisé au conducteur par serrage élastique des spires de ce dernier sur la tige dudit conducteur ;
- les spires du conducteur sont serrées sur la tige de l'organe de fixation par une bague de sertissage ;
- la tête de l'organe de fixation est munie d'une fente permettant son vissage dans les spires du conducteur ;
- l'organe de fixation est constitué par un tube creux évasé à son extrémité ;
- un moyen de fixation active est fixé à l'intérieur du tube creux ;
- l'extrémité évasée du tube creux est obturée par une membrane poreuse à travers laquelle on peut injecter ou diffuser un liquide ;
- la surface extérieure de la tige est rendue rugueuse par exemple par sablage ;
- la fixation de l'électrode est complétée par une couche d'adhésif intercalée entre la base de l'électrode et l'extrémité de la sonde ;
- la surface de l'électrode annulaire est diminuée par découpes successives ;
- la fixation de l'électrode annulaire sur l'extrémité de la sonde est complétée par interposition d'un adhésif ;
- l'axe de l'électrode annulaire est oblique par rapport à celui de l'organe de fixation ;
- l'organe de fixation est radio-opaque ;
- l'électrode annulaire est réalisée dans un matériau inorganique tel que les céramiques et les carbones ;
- l'électrode annulaire est réalisée dans un matériau métallique tel que du platine iridié.

A titre d'exemples non limitatifs et pour faciliter la compréhension de l'invention, on a représenté aux dessins annexés :
Figure 1 une vue en demi-coupe longitudinale d'un mode de réalisation de la sonde selon l'invention;
Figure 2 une vue partielle en demi-coupe d'une variante de réalisation de la figure 1 ;
Figures 3 et 4 deux vues partielles d'électrode;
Figure 5 une vue illustrant une variante de réalisation de la fixation de l'électrode ;
Figure 6 une vue illustrant une variante de réalisation de la fixation du conducteur sur la tige de fixation ;
Figure 7 une vue analogue à la figure 1, illustrant une autre variante de réalisation de la fixation de l'électrode ;
Figure 8 une vue partielle illustrant une variante de réalisation de l'organe de fixation permettant la mise en place d'un moyen de fixation active ;
Figure 9 une vue partielle illustrant une variante de réalisation de l'organe de fixation permettant l'injection de produits liquides;
Figure 10 une vue en demi-coupe longitudinale illustrant un autre mode de réalisation de l'invention.

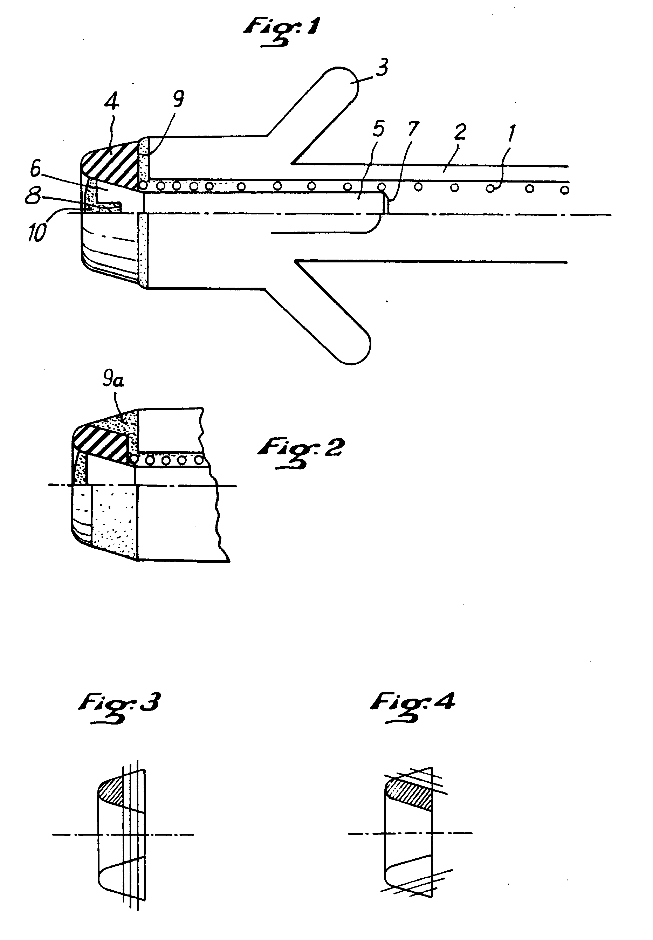

En se reportant à la figure 1, on voit que la sonde est constituée, comme cela est connu en soi, par un fil conducteur 1, enroulé en spirales qui est disposé à l'intérieur d'une gaîne souple 2, par exemple en caoutchouc au silicone, qui peut avantageusement être munie de barbules 3 également en caoutchouc au silicone.

Selon la présente invention l'électrode proprement dite, c'est-à-dire la pièce en matériau conducteur, qui doit venir en contact avec la paroi intérieure du muscle cardiaque pour transmettre au muscle cardiaque le signal électrique de stimulation, est constituée par une pièce annulaire 4 dont la section est sensiblement trapézoïdale.

Cette pièce annulaire 4 est traversée par un organe de fixation qui comporte une tige 5 qui s'engage à l'intérieur de l'enroulement 1 et une tête 6 qui assure le blocage de ladite pièce annulaire 4.

Dans l'exemple représenté à la figure 1, cette tête 6 est constituée par un tronc de cône dont la pente est égale à celle de la pente de la paroi interne de la pièce annulaire 4 de façon à bloquer fermement celle-ci en position par coincement. Un tel blocage suppose que la tige 5 soit fermement bloquée elle-même dans l'enroulement 1. A cette fin, le diamètre de la tige 5 doit être légèrement supérieur au diamètre interne dudit enroulement 1. On peut améliorer encore ce blocage en rendant rugueuse la surface de la tige 5 par tout moyen approprié, par exemple par un sablage.

L'extrémité 7 de la tige 5 constitue une butée qui, lorsque l'on emploie un mandrin pour la mise en place de la sonde, empêche ce mandrin de traverser l'extrémité distale de la sonde.

La tige 5 peut être enfoncée à force dans l'enroulement 1 ou bien y être mise en place par vissage et dans ce cas la tête 6 comporte avantageusement une fente 8 pour permettre l'emploi d'un tournevis.

De préférence, la fixation de l'électrode 4 est parachevée par l'emploi d'un adhésif 9 qui est intercalée entre la base de la pièce annulaire et l'extrémité de la sonde.

De plus, la tête 6 de l'organe de fixation est recouverte d'une couche d'une matière isolante 10, qui peut être la même matière que celle employée pour l'adhésif 9.

Cette couche de matière isolante 10 a pour effet de rendre non conductrice la partie centrale de l'électrode 4 et donc d'augmenter la densité électrique à la surface de cette dernière.

On peut encore diminuer la surface conductrice comme cela est représenté aux figures 2 à 4 en procédant à des découpes soit horizontales (figure 3), soit obliques (figure 4). Dans le cas d'une découpe latérale oblique du type représenté à la figure 4 la matière conductrice ainsi enlevée peut avantageusement être remplacée par de la matière adhésive 9a, comme représenté à la figure 2.

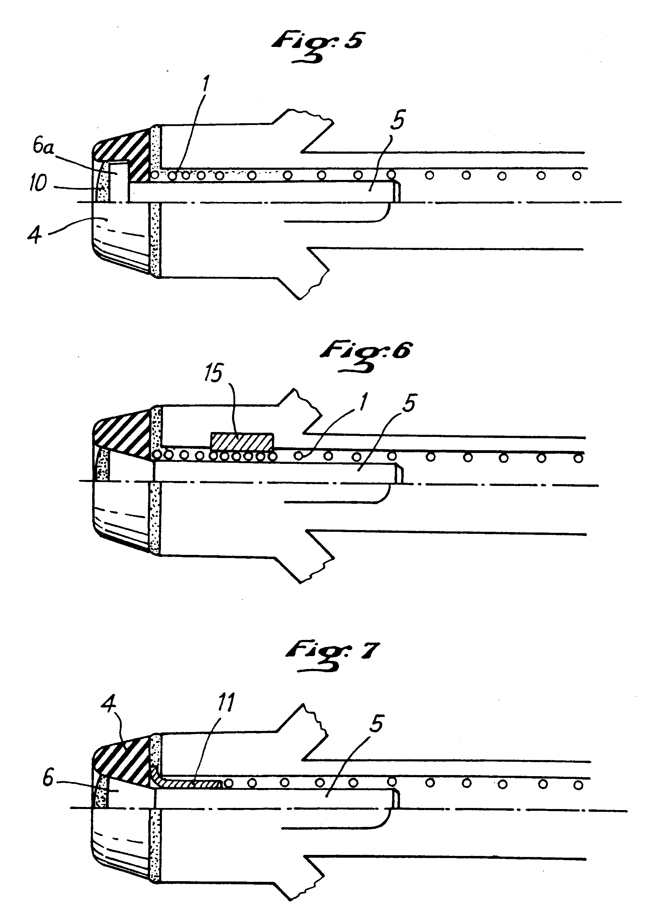

Les figures 5 et 6 et 7 représentent trois variantes de réalisation de la fixation de d'électrode annulaire 4 au moyen de l'organe de fixation 5-6.

Sur la figure 5, on voit que la tige 5 est munie d'une tête plate 6a, l'électrode 4 comportant un épaulement interne dans lequel vient s'engager ladite tête plate 6a : l'électrode 4 est alors bloquée entre la tête plate 6a et l'extrémité de l'enroulement 1.

Sur la figure 6, on voit que le conducteur 1 est maintenu fermement sur la tige 5 de l'organe de fixation par sertissage de la bague additionnelle 15.

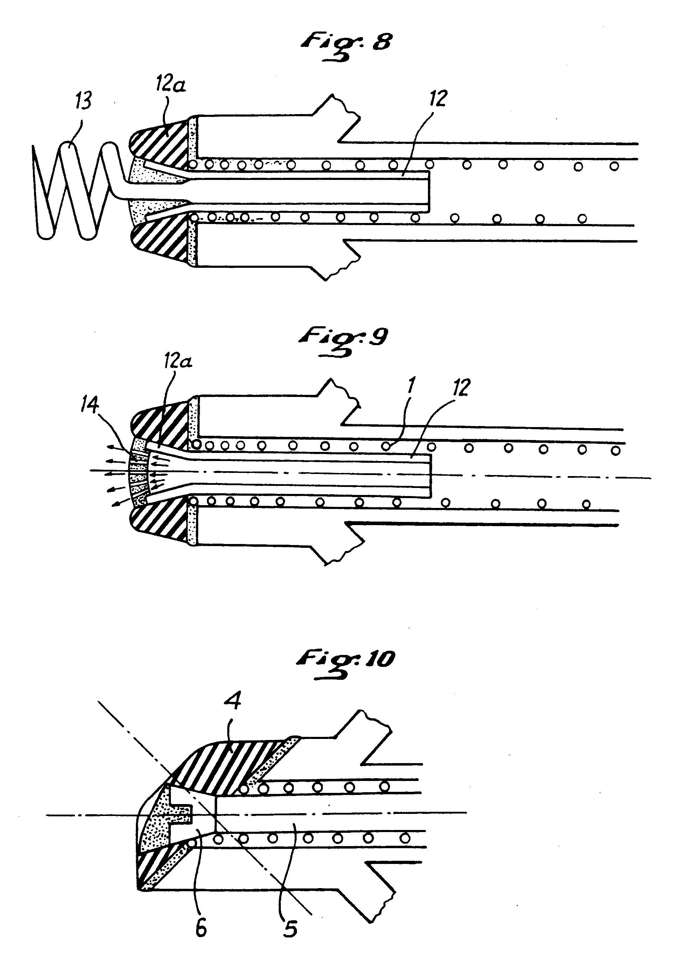

Sur la figure 7, l'électrode 4 est bloquée entre la tête 6 et une bague additionnelle 11 qui est sertie sur la tige 5.

Les figures 8 et 9 représentent deux variantes de réalisation selon lesquelles l'organe de fixation de la figure 1 est un tube creux 12 dont l'extrémité 12a est évasée. Cette extrémité 12a remplit la même fonction que la tête tronconique 6 de la figure 1, la partie cylindrique 12 remplissant la même fonction que la tige 5. Cette disposition permet comme représenté à la figure 8, la fixation, à l'intérieur de l'organe de fixation 12 d'un moyen de fixation active 13 qui peut être une pièce hélicoïdale du type tire-bouchon ou un crochet. Ces moyens de fixation active sont en eux-mêmes connus. Cette disposition permet aussi selon la variante représentée à la figure 9 d'obturer l'extrémité 12a de l'organe de fixation au moyen d'une membrane perméable 14 : en travers de laquelle on peut faire diffuser un liquide ou un gel.

La figure 10 présente une variante de l'électrode 4 selon laquelle l'axe de l'organe de fixation 5, 6 n'est pas confondu avec l'axe de l'électrode 4. En effet, celui-ci peut ne pas être concentrique à l'électrode et peut être incliné. Une telle disposition peut, par exemple, s'avérer avantageuse lorsque la sonde comporte une extrémité repliée en J pour s'appliquer contre l'apex d'une oreillette.

## Revendications

1. Sonde endocardiaque pour stimulateur cardiaque, du type constitué par un conducteur (1) enroulé en spirale, disposé à l'intérieur d'une gaine souple (2), à l'extrémité duquel est placée une électrode constituée par une pièce annulaire (4) traversée par un organe de fixation (5-6) comportant une tige (5) s'engageant à l'intérieur du conducteur (1) et une tête (6) assurant le maintien de l'électrode (4), caractérisée en ce que : l'électrode (4) est placée sur l'extrémité distale de la gaine souple (2), et est maintenue par coincement entre le conducteur (1) enroulé en spirale et la tête (6) de l'organe de fixation.

2. Sonde selon la revendication 1, dans laquelle la tête (6) est recouverte d'une couche (10) de matériau isolant.

3. Sonde selon la revendication 2, dans laquelle la tête (6) de l'organe de fixation constitue une butée dont la forme est complémentaire de l'orifice central de l'électrode annulaire (4).

4. Sonde selon la revendication 3, dans laquelle la tête (6) de l'organe de fixation est tronconique et s'adapte à la forme tronconique correspondante de l'orifice central de l'électrode annulaire (4).

5. Sonde selon la revendication 3, dans laquelle la tête (6) de l'organe de fixation est plate (6a) et prend appui sur un épaulement circulaire de forme correspondante ménagé dans l'orifice central de l'électrode annulaire (4).

6. Sonde selon la revendication 3, dans laquelle une bague (11) est sertie sur la tige (5) de l'organe de fixation de sorte que l'électrode annulaire (4) est bloquée entre la tête (6) dudit organe de fixation et la bague.

7. Sonde selon l'une quelconque des revendications précédentes, dans laquelle l'organe de fixation est solidarisé au conducteur (1) par serrage élastique des spires de ce dernier sur la tige (5) dudit conducteur (1).

8. Sonde selon l'une quelconque des revendications précédentes, dans laquelle les spires du conducteur (1) sont serrées sur la tige (5) de l'organe de fixation par une bague de sertissage (15).

9. Sonde selon l'une quelconque des revendications 7 et 8, dans laquelle la tête (6) de l'organe de fixation est munie d'une fente (5) permettant son vissage dans les spires du conducteur (1).

10. Sonde selon la revendication 3, dans laquelle l'organe de fixation (5-6) est constitué par un tube creux (12) évasé à son extrémité (12a).

11. Sonde selon la revendication 10, dans laquelle un moyen de fixation active (13) est fixé à l'intérieur du tube creux (12).

12. Sonde selon la revendication 10, dans laquelle l'extrémité évasée (12a) du tube creux (12) est obturée par une membrane poreuse (14) à travers laquelle on peut injecter ou diffuser un liquide.

13. Sonde selon l'une quelconque des revendications précédentes, dans laquelle la surface extérieure de la tige (5 ou 12) est rendue rugueuse par exemple par sablage.

14. Sonde selon l'une quelconque des revendications précédentes, dans laquelle la fixation de l'électrode (4) est complétée par une couche d'adhésif (9) intercalée entre la base de l'électrode (4) et l'extrémité de la sonde.

15. Sonde selon l'une quelconque des revendications précédentes, dans laquelle la surface de l'électrode annulaire (4) est diminuée par découpes successives.

16. Sonde selon l'une quelconque des revendications précédentes, dans laquelle la fixation de l'électrode annulaire (4) sur l'extrémité de la sonde est complétée par interposition d'un adhésif (9).

17. Sonde selon l'une quelconque des revendications précédentes, dans laquelle l'axe de l'électrode annulaire (4) est oblique par rapport à celui de l'organe de fixation (5-6).

18. Sonde selon l'une quelconque des revendications précédentes, dans laquelle l'organe de fixation (5-6) est radio-opaque.

19. Sonde selon l'une quelconque des revendications précédentes, dans laquelle l'électrode annulaire (4) est réalisée dans un matériau inorganique tel que les céramiques et les carbones.

20. Sonde selon l'une quelconque des revendications précédentes, dans laquelle l'électrode annulaire (4) est réalisée dans un matériau métallique tel que du platine iridié.

## Claims

1. An endocardial probe for cardiac pacemaker, of the type constituted by a lead (1) wound in a spiral, disposed inside a flexible sheath (2), at the end of which is placed an electrode constituted by an annular part (4) passed through by an attachment device (5-6) comprising a rod (5) engaging inside said lead (1) and a head (6) ensuring said electrode (4) is maintained, characterized in that said electrode (4) is placed at the distal end of said flexible sheath (2) and is maintained by jamming between said lead (1) wound in a spiral and said head (6) of said attachment device.

2. The probe as claimed in claim 1, wherein said head (6) is covered with a layer (10) of insulating material.

3. The probe as claimed in claim 2, wherein said head (6) of said attachment device constitutes a stop of shape complementing said central hole of said annular electrode (4).

4. The probe as claimed in claim 3, wherein said head (6) of said attachment device is tapered and matches the corresponding tapered shape of said central hole of said annular electrode (4).

5. The probe as claimed in claim 3, wherein said head (6) of said attachment device is flat (6a) and rests against a circular shoulder of corresponding shape arranged in said central hole of said annular electrode (4).

6. The probe as claimed in claim 3, wherein a ring (11) is crimped onto said rod (5) of the attachment device so that said annular electrode (4) is blocked between said head (6) of said attachment device and the ring.

7. The probe as claimed in any of the previous claims, wherein said attachment device is made interdependent with said lead (1) by elastic locking of the spires of the latter onto said rod (5) of said lead (1).

8. The probe as claimed in any one of the previous claims, wherein said spires of the lead (1) are secured to the rod (5) of said attachment device by a crimping ring (15).

9. The probe as claimed in either claim 7 or 8, wherein said head (6) of said attachment device is fitted with a slot (8) enabling it to be screwed into said spires of said lead (1).

10. The probe as claimed in claim 3, wherein said attachment device (5-6) is constituted by a hollow tube (12) flared at its end (12a).

11. The probe as claimed in claim 10, wherein an active attachment means (13) is fixed inside said hollow tube (12).

12. The probe as claimed in claim 10, wherein the flared end (12a) of said hollow tube (12) is sealed by a porous membrane (14) through which a liquid can be injected or diffused.

13. The probe as claimed in any one of the previous claims, wherein the outer surface of said rod (5 or 12) is roughened e.g. by sand blasting.

14. The probe as claimed in any one of the previous claims, wherein the attachment of said electrode (4) is completed by an adhesive layer (9) interposed between the base of said annular electrode (4) and the end of said probe.

15. The probe as claimed in any one of the previous claims, wherein the surface of said annular electrode (4) is diminished by successive cuttings.

16. The probe as claimed in any one of the previous claims, wherein the attachment of said annular electrode (4) to said probe end is completed by the interposing of an adhesive.

17. The probe as claimed in any one of the previous claims, wherein the axis of said annular electrode (4) is oblique with regard to that of said attachment device (5-6).

18. The probe as claimed in any one of the previous claims, wherein said attachment device (5-6) is radiopaque.

19. The probe as claimed in any one of the previous claims, wherein said annular electrode (4) is manufactured in inorganic material such as ceramics and carbons.

20. The probe as claimed in any one of the previous claims, wherein said annular electrode (4) is manufactured in a metallic material such as platinum iridium.

## Patentansprüche

1. Endokardial-Sonde für einen Herzstimulator, bestehend aus einem spiralförmigen Leiter (1), der im Inneren einer elastischen Hülle (2) angeordnet ist und an dessen Ende sich eine Elektrode befindet, die aus einem ringförmigen Teil (4) besteht, durch welches sich ein Befestigungselement (5-6) mit einem mit dem Inneren des Leiters (1) in Eingriff stehenden Schaft (5) und einem den Halt der Elektrode sichernden Kopf (6) hindurchzieht,
**dadurch gekennzeichnet, daß**
die Elektrode (4) an dem entfernten Ende der elastischen Hülle (2) angebracht ist und durch Verkeilung zwischen dem spiralförmigen Leiter (1) und dem Kopf (6) des Befestigungselementes gehalten wird.

2. Eine Sonde gemäß Anspruch 1, deren Kopf (6) mit einer Schicht (10) Isoliermaterial überzogen ist.

3. Eine Sonde gemäß Anspruch 2, bei der der Kopf (6) des Befestigungselementes einen Anschlag bildet, dessen Form der zentralen Öffnung der ringförmigen Elektrode (4) entspricht.

4. Eine Sonde gemäß Anspruch 3, bei der der Kopf (6) des Befestigungselementes kegelstumpfartig ist und sich an die entsprechende kegelstumpfartige Form der zentralen Öffnung der ringförmigen Elektrode (4) anpaßt.

5. Eine Sonde gemäß Anspruch 3, bei der der Kopf (6) des Befestigungselementes flach ist (6a) und sich auf eine entsprechende kreisförmige Schulter stützt, die in der zentralen Öffnung der kreisförmigen Elektrode (4) ausgebildet ist.

6. Eine Sonde gemäß Anspruch 3, in der ein Ring (11) auf den Schaft (5) des Befestigungselementes so aufgesetzt ist, daß die ringförmige Elektrode (4) zwischen dem Kopf (6) des genannten Befestigungselementes und dem Ring gesichert ist.

7. Eine Sonde gemäß einem der vorangegangenen Ansprüche, bei der das Befestigungselement mit dem Leiter (1) durch flexibles Befestigen der Windungen des letzteren am Schaft (5) des Leiters (1) verbunden wird.

8. Eine Sonde gemäß einem der vorangegangenen Ansprüche, bei der die Windungen des Leiters (1) am Schaft (5) des Befestigungselementes mittels eines Verbindungsringes (15) befestigt sind.

9. Eine Sonde gemäß einem der vorangegangenen Ansprüche 7 und 8, bei der der Kopf (6) des Befestigungselementes mit einem Schlitz (5) zur Verschraubung in den Windungen des Leiters (1) versehen ist.

10. Eine Sonde gemäß Anspruch 3, bei der das Befestigungselement (5-6) aus einem hohlen, an seinem Ende (12a) erweiterten Rohr (12) besteht.

11. Eine Sonde gemäß Anspruch 10, bei der ein aktives Verbindungsmittel (13) im Inneren des hohlen Rohres (12) befestigt ist.

12. Eine Sonde gemäß Anspruch 10, bei der das erweiterte Ende (12a) des hohlen Rohres (12) mit einer porösen Membran (14) verschlossen ist, durch die eine Flüssigkeit eingespritzt oder zerstreut werden kann.

13. Eine Sonde gemäß einem der vorangegangenen Ansprüche, bei der die Außenfläche des Schaftes (5 oder 12) z.B. durch Sandstrahlen angerauht wird.

14. Eine Sonde gemäß einem der vorangegangenen Ansprüche, bei der die Befestigung der Elektrode (4) durch eine zwischen der Elektrodenbasis (4) und dem Ende der Sonde eingefügte Schicht Kleber (9) vervollständigt wird.

15. Eine Sonde gemäß einem der vorangegangenen Ansprüche, bei der die Oberfläche der ringförmigen Elektrode (4) durch aufeinanderfolgende Nuten verkleinert wird.

16. Eine Sonde gemäß einem der vorangegangenen Ansprüche, bei der die Befestigung der ringförmigen Elektrode (4) an dem Ende der Sonde durch Zwischenlage eines Klebers (9) vervollständigt wird.

17. Eine Sonde gemäß einem der vorangegangenen Ansprüche, bei der die Achse der ringförmigen Elektrode (4) quer zu der des Befestigungselementes (5-6) verläuft.

18. Eine Sonde gemäß einem der vorangegangenen Ansprüche, bei der das Befestigungselement (5-6) radio-opak ist.

19. Eine Sonde gemäß einem der vorangegangenen Ansprüche, bei der die ringförmige Elektrode (4) aus einem anorganischen Material, z.B. Keramiken und Kohlenstoffen, gebildet wird.

20. Eine Sonde gemäß einem der vorangegangenen Ansprüche, bei der die ringförmige Elektrode (4) aus einem metallischen Material, z.B. Platiniridium, gebildet wird.
